(19) Europäisches Patentamt
European Patent Office
Office européen des brevets

(11) Publication number: **0 181 721 B1**

(12) # EUROPEAN PATENT SPECIFICATION

(45) Date of publication of patent specification: **02.08.89**

(51) Int. Cl.⁴: **C 07 F 5/02,** A 61 K 31/69

(21) Application number: **85307732.9**

(22) Date of filing: **25.10.85**

(54) **Esters of boron analogues of amino acids.**

(30) Priority: **25.10.84 US 664647**

(43) Date of publication of application:
**21.05.86 Bulletin 86/21**

(45) Publication of the grant of the patent:
**02.08.89 Bulletin 89/31**

(84) Designated Contracting States:
**AT BE CH DE FR GB IT LI NL**

(56) References cited:
**EP-A-0 034 238**
**US-A- 312 989**

**CHEMICAL ABSTRACTS, vol. 102, no. 3, 21st January 1985, page 701, no. 24676a, Columbus, Ohio, US; B.F. SPIELVOGEL et al.: "Boron analogs of amino acids. 4. Synthesis of glycine and N-methylated glycine ester analogs", & INORG. CHEM. 1984, 23(25), 4322-4**

**MOL. CRYST. LIQ. CRYST., vol. 128, 1985, pages 65-73, Gordon and Breach Science Publishers Inc. and OPA Ltd., US; H. HAKEMI et al.: "Investigation of mixtures of cholesteryl esters of boron analogues of amino acids with p-azoxyanisole"**

(73) Proprietor: **DUKE UNIVERSITY**
**2111 Campus Drive**
**Durham, NC 27706 (US)**

(72) Inventor: **Spielvogel, Bernard F.**
**2811 O'Berry Street**
**Raleigh North Carolina 27607 (US)**
Inventor: **McPhail, Andrew T.**
**331 Smith Drive**
**Durham North Carolina 22712 (US)**
Inventor: **Hall, Iris H.**
**328 Azaela Drive**
**Chapel Hill North Carolina 27514 (US)**

(74) Representative: **Goldin, Douglas Michael et al**
**J.A. KEMP & CO. 14, South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 181 721 B1

**Description**

This invention relates to boron analogues of amino acids, to methods of forming esters of boron analogues of amino acids and to the use of boron analogues of amino acids as a biologically active material for antitumour or hypolipidemic activity.

Boron analogues of amino acids are broadly known in the art. The antihyperlipidemic activity of amino cyanoboranes is discussed in Antihyperlipidemic Activity of Amino Cyanoboranes, Amino Carboxyboranes and Related Compounds, *Journal of Pharmaceutical Sciences*, Vol. 70, No. 3, March 1981.

US Patents related to boron analogues of amino acids include Nos. 4,209,510, 4,312,989 and 4,368,194.

This invention provides an amine borane ester compound of general formula I:

$$R_1R_2R_3NBH_2COOR_4$$

wherein $R_4$ is $C_1$—$C_8$ alkyl, $C_1$—$C_8$ haloalkyl, phenyl, benzyl or cholesteryl and

(a) each of $R_1$, $R_2$, and $R_3$, which may be the same or different, is hydrogen, $C_1$—$C_8$ alkyl, $C_1$—$C_8$ haloalkyl, phenyl or benzyl with the proviso that at least one of $R_1$, $R_2$, and $R_3$ is hydrogen, or

(b) each of $R_1$, $R_2$, and $R_3$ is methyl with the proviso that $R_4$ is also methyl.

The alkyl groups can be straight or branched chains. Representative alkyl groups include methyl, ethyl, propyl and n-butyl. In general, the preferred moieties are methyl and ethyl groups because these moieties have minimal steric hindrance during synthesis. In the new compounds it is preferred that $R_1$ is hydrogen and $R_2$ and $R_3$ are both methyl while $R_4$ can be methyl or ethyl or possibly haloalkyl, phenyl, benzyl or cholesteryl.

Compounds of this invention as well as related compounds can be prepared by condensing the corresponding acids and alcohols with dicyclohexylcarbodiimide (DCC) at room temperature in dichloromethane and using a method disclosed hereinafter, using chloroformates, both methods forming part of the present invention.

The formation of amino carboxyborane esters as described above has provided a technique for making sufficient quantities of material for testing. However, the reaction times for the production of the compounds by condensation of the corresponding acids and alcohols with DCC at room temperature in $CH_2Cl_2$ are very long. The reaction generally gives good to moderate yields but is time consuming, i.e., up to a week or more. Moreover, the esters contain dicyclohexylurea as a by-product which must be removed. Separation of the desired product by fractional crystallisation and solvent extraction is tedious and difficult owing to the similar solubility characteristics of the desired ester and the by-products.

An alternative synthesis technique for making the ester is the treatment of trialkylaminecarboxyborane with a tetrafluoroborate compound, as disclosed in US Patent 4,368,194, was effective for compounds where the initial carboxyborane had a triamine substituent. It does not work with carboxyboranes of diamines, monoamine or ammonia since the hydrogens on the boron in the borane hydrolyse.

A new and more efficient synthesis with a more general application is disclosed in this Application. The new synthesis can be used to produce esters regardless of the presence or absence of hydrogen atoms on the nitrogen of the amine carboxyborane. In general, the desired esters are formed by the reaction of mixed carboxylic-carbonic anhydrides under mild conditions. In the improved synthesis, mixing equimolar amounts of aminecarboxyborane, alkylchloroformate, and trialkylamine in methylene chloride at reduced temperatures i.e., −10 to +10°C produces a rapid decarboxylation to give the desired ester product.

As an example, 0.01 mole of alkylchloroformate and 0.001 mole of dimethylaminopyridine were added to a solution of 0.01 mole of amine - carboxyborane and 0.011 mole of triethylamine in 100 ml of methylene chloride. The mixture was maintained at 0°C for one hour with constant stirring. The reaction proceeded smoothly with the evolution of carbon dioxide. After one hour, the solution was given two washings with 20 ml of water and dried using magnesium sulphate. The resulting material was concentrated to pure ester.

Using various starting materials, esters of the basic configuration $(R)_3N \cdot BH_2COOR'$ were made using various starting compounds. Esters were formed where R was methyl or ethyl. The number of organic moieties attached to the amino nitrogen was varied from 1 to 3.

Esters were formed where R' was methyl, ethyl, cholesteryl, toluene, benzene, bromoethyl and chloroethyl.

This method allows a rapid production at high yields of a wide variety of ester materials.

A further aspect of the present invention provides a pharmaceutical composition for lowering of serum cholesterol or triglyceride level which comprises a therapeutically effective amount of at least one of the amine - carboxyborane esters of formula I and a pharmaceutically acceptable carrier. The invention also extends to the esters of formula I and the compositions containing them for use in a method of surgery or therapy on the human or animal body or in a method of diagnosis practised on the human or animal body.

The following Examples are given to illustrate the invention.

Example 1

Trimethylamine - carbethoxyborane $(CH_3)_3N \cdot BH_2COOC_2H_5$, was prepared by dehydrating a solution of $(CH_3)_3N \cdot BH_2COOH$ and absolute ethanol with DCC at room temperature for 6 days. This procedure

resulted in a 45% yield. The relatively high volatility and solubility in water of this sweet-smelling ester probably contributed to its low yield in this procedure.

Example 2

Trimethylamine - carbomethoxyborane $(CH_3)_3N \cdot BH_2COOCH_3$ was prepared with an 82% yield by condensing $(CH_3)_3N \cdot BH_2COOH$ and $CH_3OH$ with DCC at room temperature for one week; extension of the reaction period to two weeks led to an increase in the yield to 98%.

Example 3

Dimethylamine - carbomethoxyborane $(CH_3)_3NH \cdot BH_2COOCH_3$, was prepared in 67% yield by an amino-exchange reaction of $(CH_3)_3N \cdot BH_2COOCH_3$ with an 8-fold excess (by weight) of $(CH_3)_2NH$ in a glass pressure reaction vessel for 2 weeks at room temperature. The 8% unreacted starting ester in the product mixture was removed by washing with $H_2O$ and vacuum pumping. The ester linkages in the starting material and product were not cleaved by the excess amine. As an alternative, condensing $(CH_3)_2NH \cdot BH_2COOH$ and $CH_3OH$ with DCC at room temperature for 4 days could be performed. This reaction procedure gave a very low yield of 8%.

Example 4

Methylamine - carbomethoxyborane, $CH_3NH_2 \cdot BH_2COOCH_3$ was prepared by condensing $CH_3NH_2 \cdot BH_2COOH$ and $CH_3OH$ with DCC at room temperature for 6 days. This reaction had a 21% yield.

Example 5

Trimethylamine - (carbo - 2 - chloroethoxy)borane, $(CH_3)_3N \cdot BH_2COOCH_2CH_2Cl_{(s)}$, was prepared in a manner similar to the preparation of the compound of Example 4 by condensing $(CH_3)_3N \cdot BH_2COOH$ and $HOCH_2CH_2Cl$ with DCC at room temperature for 1 week. This reaction yielded 61%.

Example 6

Ammonia - carbomethoxyborane, $H_3N \cdot BH_2COOCH_3$, was prepared by an amine-exchange reaction carried out in a stainless steel pressure vessel between $(CH_3)_3N \cdot BH_2COOCH_3$ and excess liquid $NH_3$ at room temperature for 2 weeks.

All of these compounds were characterised by elemental analysis and IR, H NMR and B NMR spectroscopy. Physical and spectral data of the esters are given in Table I. IR spectra exhibited characteristic B-H and C=0 absorptions; H and B NMR spectral data were consistent with the structures shown for the compounds. IR spectra were recorded on a Perkin-Elmer 297 spectrometer. Solid samples were prepared as KBr disks, as Nujol mulls between NaCl disks, or as solutions in suitable solvents; oils were recorded neat. Proton and boron NMR spectra were obtained on Varian EM 360A and Jeol FX 90Q spectrometers, respectively. Elemental analyses were performed by Galbraith Laboratories Inc., Knoxville, TN, or Schwarzkopf Microanalytical Laboratory Inc., Woodside, NY.

TABLE 1

| Compd. | Ester | bp/mp°C | Yield % | Shifts ppm[a] | $J_{B-11}$ · Hz |
|---|---|---|---|---|---|
| 1 | $(CH_3)_3N \cdot BH_2COOC_2H_5$ | 45—47 | 34—41 | −9.17*(t) | 98 |
| 2 | $(CH_3)_3N \cdot BH_2COOCH_3$ | 90—92 | 82—98 | −9.09 (t) | 99 |
| 3 | $(CH_3)_2NH \cdot BH_2COOCH_3$ | 52—53 | 67 | −12.57 (t) | 95 |
| 4 | $CH_3NH_2BH_2COOCH_3$ | 56—57 | 21 | −16.22 (t) | 98 |
| 5 | $(CH_3)_3N \cdot BH_2COOCH_2CH_2Cl$ | | 61 | −8.75 | 97 |
| 6 | $H_3N \cdot BH_2COOCH_3$ | 92—93 | 49 | −20.45 (t) | 94 |

[a]$(C_2H_5)_2O \cdot BF_3$ was used as an external standard; all chemical shifts reported here were (negative) upfield from the standard.

Serum lipid screening

$CF_1$ male mice (30 g) were fed rodent laboratory food with water *ad libitum* during the experiment. The compounds of this invention were suspended in 1% carboxymethylcellulose - water and homogenized. The doses were calculated on the weekly weights of the mice. Test compounds were administered at a rate of 20 mg/kg/day ip. On the 9th and 16th days, blood was collected by tail vein bleeding in alkali-free, non-heparinised microcapillary tubes and centrifuged 3 minutes to obtain the serum. Duplicate 30-ml samples of non-hemolysed serum were used to determine the serum cholesterol levels (milligram percent)

by a modification of the Liebermann-Burchard reaction described in Ness *et al, Clin, Chem, Acta* Vol. 10, page 229 (1964). A separate group of mice were bled on day 14, and their serum triglyceride levels (milligram percent) were determined by using 25-ml samples.

The results of the serum testing are set out in Table 2. The values given are percent control. The percentage inhibition, the effectiveness of the compound, is determined by subtracting the control percentage from 100.

TABLE 2
Percentage control of cholesterol and triglyceride

| Compound | I.P. Dose | Serum cholesterol Day 9 | Serum cholesterol Day 16 | Serum triglyceride Day 16 |
|---|---|---|---|---|
| $MeNH_2BH_2COOMe$ | 8 | 69 | — | — |
| $Me_3NBH_2COOMe$ | 8 | 79 | 58 | 23 |
| $Me_2NHBN_2COOMe$ | 8 | 73 | 68 | 69 |

All of the compounds show a degree of control on both serum cholesterol and triglycerides. In particular trimethylaminecarbomethoxyborane showed an inhibition effect of 77% on serum triglycerides. There is a significant increase in activity over the previously reported activity of the corresponding ethyl ester which showed an inhibition of only 43% at a dosage of 20 mg/kg as reported in J. Pharm. Sci. *70*, 339, (1981).

**Claims**

1. An amine borane ester compound of general formula I:

$$R_1R_2R_3NBH_2COOR_4$$

wherein $R_4$ is $C_1$—$C_8$ alkyl, $C_1$—$C_8$ haloalkyl, phenyl, benzyl or cholesteryl and

(a) each of $R_1$, $R_2$, and $R_3$, which may be the same or different is hydrogen, $C_1$—$C_8$ alkyl, $C_1$—$C_8$ haloalkyl, phenyl or benzyl with the proviso that at least one of $R_1$, $R_2$, and $R_3$ is hydrogen, or

(b) each of $R_1$, $R_2$, and $R_3$ is methyl with the proviso that $R_4$ is also methyl.

2. A compound according to claim 1 wherein $R_1$ is hydrogen and $R_2$ and $R_3$ are each methyl or ethyl.

3. A compound according to claim 1 or 2 wherein $R_4$ is methyl or ethyl.

4. A compound according to claim 1 or 2 wherein $R_4$ is haloalkyl.

5. A compound according to claim 1 or 2 wherein $R_4$ is cholesteryl.

6. A compound according to claim 1 or 2 wherein $R_4$ is phenyl.

7. A compound according to claim 1 or 2 wherein $R_4$ is benzyl.

8. A compound according to claim 1 wherein each of $R_1$, $R_2$, $R_3$ and $R_4$ is methyl.

9. A method of forming an amine borane ester comprising the steps of: mixing equimolar amounts of an amine - carboxyborane and an aryl- or alkylchloroformate and at least an equimolar amount of a trialkylamine in a compatible solvent; and isolating the amine carboxyborane ester from the reaction mixture.

10. A method according to claim 9 wherein a catalytic amount of dimethylaminopyridine is added to the reaction mixture.

11. A method according to claim 9 or 10 wherein the aryl- or alkylchloroformate has the formula ClCOOR wherein R is a $C_1$—$C_8$ alkyl, $C_1$—$C_8$ haloalkyl, phenyl, benzyl or cholesteryl group.

12. A method according to any one of claims 9 to 11 wherein the amine carboxyborane has the formula

$$R_1R_2R_3NBH_2COOH$$

wherein each of $R_1$, $R_2$ and $R_3$ is hydrogen or a $C_1$—$C_8$ alkyl, $C_1$—$C_8$ haloalkyl, phenyl or benzyl group.

13. A method according to any one of claims 9 to 12 wherein the trialkylamine is trimethylamine or triethylamine.

14. A method according to any one of claims 9 to 13 wherein the solvent is methylene chloride.

15. A method according to any one of claims 9 to 14 wherein the reaction is carried out at a temperature of from −10°C to +10°C.

16. A method of forming an amine borane ester, which comprises condensing an amine borane carboxylic acid with an alcohol in the presence of dicyclohexylcarbodiimide.

17. A method according to claim 16 wherein said condensing occurs in excess alcohol as a solvent.

18. A method according to claim 16 wherein said condensing occurs in a solution of $CH_2Cl_2$.

19. A pharmaceutical composition for the lowering of serum cholesterol or triglyceride level, which comprises a therapeutically effective amount of a compound according to any one of claims 1 to 8 and a pharmaceutically acceptable carrier.

20. A compound according to any one of claims 1 to 8 or composition according to claim 19 for use in a method of treatment of the human or animal body by surgery or therapy or in a method of diagnosis practised on the human or animal body.

**Patentansprüche**

1. Aminboranesterverbindung der allgemeinen Formel (I)

$$R_1R_2R_3NBH_2COOR_4,$$

worin $R_4$ $C_1$—$C_8$-Alkyl, $C_1$—$C_8$-Halogenalkyl, Phenyl, Benzyl oder Cholesteryl bedeutet und

(a) jedes von $R_1$, $R_2$ und $R_3$, die gleich oder verschieden sein können, Wasserstoff, $C_1$—$C_8$-Alkyl, $C_1$—$C_8$-Halogenalkyl, Phenyl oder Benzyl ist, mit der Maßgabe, daß mindestens eines von $R_1$, $R_2$ und $R_3$ Wasserstoff darstellt, oder

(b) jedes von $R_1$, $R_2$ und $R_3$ Methyl ist, mit der Maßgabe, daß $R_4$ ebenfalls Methyl ist.

2. Verbindung nach Anspruch 1, worin $R_1$ Wasserstoff bedeutet und $R_2$ und $R_3$ jeweils Methyl oder Äthyl sind.

3. Verbindung nach Anspruch 1 oder 2, worin $R_4$ Methyl oder Äthyl bedeutet.

4. Verbindung nach Anspruch 1 oder 2, worin $R_4$ Halogenalkyl bedeutet.

5. Verbindung nach Anspruch 1 oder 2, worin $R_4$ Cholesteryl bedeutet.

6. Verbindung nach Anspruch 1 oder 2, worin $R_4$ Phenyl bedeutet.

7. Verbindung nach Anspruch 1 oder 2, worin $R_4$ Benzyl bedeutet.

8. Verbindung nach Anspruch 1, worin jedes von $R_1$, $R_2$, $R_3$ und $R_4$ Methyl bedeutet.

9. Verfahren zum Herstellen eines Aminboranesters umfassend folgende Schritte: Mischen äquimolarer Mengen eines Amincarboxyborans und eines Aryl- oder Alkylchlorformiats und mindestens einer äquimolaren Menge eines Trialkylamins in einem verträglichen Lösungsmittel und Isolieren des Amincarboxyboranesters von der Reaktionsmischung.

10. Verfahren nach Anspruch 9, worin eine katalytische Menge Dimethylaminopyridin der Reaktionsmischung zugesetzt wird.

11. Verfahren nach Anspruch 9 oder 10, worin das Aryl- oder Alkylchlorformiat die Formel ClCOOR aufweist, worin R eine $C_1$—$C_8$-Alkyl-, $C_1$—$C_8$-Halogenalkyl-, Phenyl-, Benzyl- oder Cholesterylgruppe bedeutet.

12. Verfahren nach einem der Ansprüche 9 bis 11, worin das Amincarboxyboran die Formel

$$R_1R_2R_3NBH_2COOH$$

aufweist, worin jedes $R_1$, $R_2$ und $R_3$ Wasserstoff oder eine $C_1$—$C_8$-Alkyl-, $C_1$—$C_8$-Halogenalkyl-, Phenyl- oder Benzylgruppe bedeutet.

13. Verfahren nach einem der Ansprüche 9 bis 12, worin das Trialkylamin, Trimethylamin oder Triäthylamin ist.

14. Verfahren nach einem der Ansprüche 9 bis 13, worin das Lösungsmittel Methylenchlorid ist.

15. Verfahren nach einem der Ansprüche 9 bis 14, worin die Reaktion bei einer Temperatur von −10 bis +10°C durchgeführt wird.

16. Verfahren zum Herstellen eines Aminboranesters, welches das Kondensieren einer Aminborancarbonsäure mit einem Alkohol in Anwesenheit von Dicyclohexylcarbodiimid umfaßt.

17. Verfahren nach Anspruch 16, worin dieses Kondensieren in überschüssigem Alkohol als Lösungsmittel erfolgt.

18. Verfahren nach Anspruch 16, worin dieses Kondensieren in einer $CH_2Cl_2$-Lösung erfolgt.

19. Pharmazeutische Zusammensetzung zum Senken des Serumcholesterin- oder Triglyceridspiegels, welche eine therapeutisch wirksame Menge einer Verbindung nach einem der Ansprüche 1 bis 8 und einen pharmazeutisch annehmbaren Träger umfaßt.

20. Verbindung nach einem der Ansprüche 1 bis 8 oder Zusammensetzung nach Anspruch 19 zur Verwendung in einem Verfahren zur Behandlung des menschlichen oder tierischen Körpers durch Operation oder Therapie oder in einer am menschlichen oder tierischen Körper durchgeführten Diagnosemethode.

**Revendications**

1. Ester dérivé de borane aminé, de formule générale I:

$R_1R_2R_3NBH_2COOR_4$

dans laquelle $R_4$ représente un groupe alkyle en $C_1$—$C_8$, haloalkyle en $C_1$—$C_8$, phényle, benzyle ou cholestéryle; et

(a) chacun des groupes $R_1$, $R_2$ et $R_3$ qui peuvent être identiques ou différents, représente un atome d'hydrogène, un groupe alkyle en $C_1$—$C_8$, haloalkyle en $C_1$—$C_8$, phényle ou benzyle, à condition qu'au moins l'un des groupes $R_1$, $R_2$ et $R_3$, représente un atome d'hydrogène; ou

(b) chacun des groupes $R_1$, $R_2$ et $R_3$ représente un groupe méthyle, à condition que $R_4$ représente également un groupe méthyle.

2. Composé selon la revendication 1, dans lequel $R_1$ représente un atome d'hydrogène, et $R_2$ et $R_3$ représentent chacun un groupe méthyle ou éthyle.

3. Composé selon la revendication 1 ou 2, dans lequel $R_4$ représente un groupe méthyle ou éthyle.

4. Composé selon la revendication 1 ou 2, dans lequel $R_4$ représente un groupe haloalkyle.

5. Composé selon la revendication 1 ou 2, dans lequel $R_4$ représente un groupe chlolestéryle.

6. Composé selon la revendication 1 ou 2, dans lequel $R_4$ représente un groupe phényle.

7. Composé selon la revendication 1 ou 2, dans lequel $R_4$ représente un groupe benzyle.

8. Composé selon la revendication 1, dans lequel chacun des groupes $R_1$, $R_2$, $R_3$ et $R_4$, représente un groupe méthyle.

9. Procédé de préparation d'un ester dérivé de borane aminé, dans lequel on mélange des quantités équimolaires d'un amino - carboxyborane et d'un chloroformate d'aryle ou d'alkyle, et au moins une quantité équimolaire d'un trialkylamine, dans un solvant compatible; et on isole l'ester dérivé d'amino - carboxyborane, du mélange réactionnel.

10. Procédé selon la revendication 9, dans lequel on ajoute une quantité catalytique de diméthylamino-pyridine dans le mélange réactionnel.

11. Procédé selon la revendication 9 ou 10, dans lequel le chloroformate d'aryle ou d'alkyle, correspond à la formule ClCOOR, dans laquelle R représente un groupe alkyle en $C_1$—$C_8$, haloalkyle en $C_1$—$C_8$, phényle, benzyle ou cholestéryle.

12. Procédé selon l'une quelconque des revendications 9 à 11, dans lequel l'amino - carboxyborane, correspond à la formule

$R_1R_2R_3NBH_2COOH$

dans laquelle chacun des groupes $R_1$, $R_2$ et $R_3$, représente un atome d'hydrogène ou un groupe alkyle en $C_1$—$C_8$, haloalkyle en $C_1$—$C_8$, phényle ou benzyle.

13. Procédé selon l'une quelconque des revendications 9 à 12, dans lequel la trialkylamine est la triméthylamine ou la triéthylamine.

14. Procédé selon l'une quelconque des revendications 9 à 13, dans lequel le solvant est le chlorure de méthylène.

15. Procédé selon l'une quelconque des revendications 9 à 14, dans lequel la réaction est effectuée à une température de $-10°C$ à $+10°C$.

16. Procédé de préparation d'un ester dérivé de borane aminé, dans lequel on condense un acide carboxylique dérivé de borane aminé, avec un alcool, en présence de dicyclohexylcarbodiimide.

17. Procédé selon la revendication 16, dans lequel la condensation est effectuée dans un alcool en excès comme solvant.

18. Procédé selon la revendication 16, dans lequel la condensation est effectuée dans une solution à base de $CH_2Cl_2$.

19. Composition pharmaceutique pour réduire les teneurs sériques en cholestérol ou en triglycérides, comprenant une quantité efficace sur le plan thérapeutique d'un composé selon l'une quelconque des revendications 1 à 8, ainsi qu'un véhicule pharmaceutiquement acceptable.

20. Composé selon l'une quelconque des revendications 1 à 8, ou composition selon la revendication 19, destiné à être utilisé dans un procédé de traitement du corps humain ou animal par chirurgie ou thérapie, ou dans un procédé de diagnostic mis en oeuvre sur le corps humain ou animal.